# EUROPEAN PATENT APPLICATION

(11) **EP 2 857 025 A1**
(43) Date of publication of application: **08.04.2015**
(21) Application number: 13004755.8
(22) Date of filing: 02.10.2013
(51) Int. Cl.: A61K 33/00, A61P 17/02

(54) **Wound treatment composition**

(71) Applicant: Special Water Patents B.V., 3905 KZ Veenendaal (NL)
(72) Inventor: De Rijk, Jan, 3905 KZ Veenendaal (NL)
(74) Representative: Huygens, Arthur Victor

(57) **Abstract**

A wound treatment composition is provided preferably comprising (a) sodium meta-silicate, (b) sodium carbonate, (c) sodium gluconate, (d) potassium aluminum sulfate, and optionally (e) sodium chloride, which composition is especially suitable as a rinsing or soaking agent for cleaning and decontaminating surfaces of a wound, for removing biofilms from wound and other body surfaces, for dissolving incrustations or scabs from body surfaces and as a dissolving agent for dressings. The composition has a beneficial and synergistic effect on the traditional treatment of wounds with antibiotics or oxidizing agents in that it makes the latter treatment more effective.

## Description

The present invention relates to a novel wound treatment composition which is especially suitable as an rinsing agent for decontaminating surfaces of the body, for dissolving incrustations or scabs from body surfaces and as a dissolving agent for dressings.

It is well known in the art of dermatology and wound treatment that, especially in the case of chronic wounds, healing of wounds is strongly delayed by coatings on the wound. These coatings usually consist of wound exudate residues, of shed or enlarged fibrin coatings, necrotic tissue and cell debris. They offer favorable growth conditions for germs which, when pathogenic, may result in infection of the wound.

The biofilm mode of growth of the infecting organisms is one of the obstacles to the healing of chronic wounds (Wolcott and Rhoads, 2008). By definition, biofilms are microbial populations that are attached to a surface, or to the surfaces of other organisms, and encase themselves in hydrated extracellular polymeric substance (EPS), which is also referred to as "slime". The chemical and physical properties of EPS will vary, but it is mainly composed of polysaccharides. EPS is also associated with other macromolecules such as proteins, DNA, lipids, and even humic substances (Nielsen et al., 1996, Tsuneda et al., 2003).

Even in the absence of pathogenic germs, it is extremely important for promoting and accelerating wound healing to remove coatings on the wound and thoroughly clean and disinfect the wound. Adhesions to the wound and resulting pain can be avoided by timely and thorough removal of wound coatings.

Various wound-treatment agents are known which on the one hand clean a wound and remove wound coatings and on the other hand kill off germs, without acting in an allergizing, sensitizing or tissue-damaging manner.

WO 03/004013 discloses a wound treatment agent that contains, in aqueous solution, polyhexamethylene biguanide ("PHMB") and at least one surfactant which is a glycine derivative, in particular a betaine, and/or a sulfosuccinate and/or an amide based on an unbranched fatty acid.

WO 94/27440 describes anti-infective PHMB agents with a mean molecular weight Mw of 2,900 to 15,000, in particular 3,200 to 5,000, which are used as an injury antiseptic and/or injury-treatment agent or as an antimicrobial, antiviral and/or antiparasitic agent, preferably by intravenous administration. Compared with previously known PHMB disinfectants, the PHMB disclosed here has a higher mean molecular weight Mw and exhibits an enhanced microbicidal action with lower toxicity. The anti-infective agent disclosed in WO 94/27440 exhibits reduced tissue damage as compared to other anti-infective agents, but its cleaning effect is low.

WO 2011/117384 describes a wound dressing comprising a polymer substrate and a composition comprising a) at least one antimicrobial active agent and b) an agent that reduces the cytotoxicity, comprising an oil-in-water emulsion that additionally has one or more alkanediols and/or one or more glyceryl ethers.

EP-A-1103264 describes a tissue cell growth-promoting solution comprising water-containing active oxygen as a prime ingredient which promotes the reconstruction of tissues, a process that corresponds to the last of the four main steps involved in wound healing biochemical processes: "blood vessel reaction", "blood vessel coagulation", "inflammation" and "reconstruction of tissues", which would otherwise have to rely on the natural healing power of the biobody itself.

EP-A-0601891 describes an antifungal and skin healing promoting agent comprising a hypochlorite, a sulfite, a nitrite, a chlorate, hydrogen peroxide, ozone water, a nitrate, and water.

WO 2007/099398 discloses compositions comprising (a) metasilicate, (b) carbonate, (c) gluconate, and (d) sulfate, in particular potassium aluminum sulfate, for the treatment of human skin to alleviate the symptoms of cosmetic or dermatologic skin conditions, including acne, rosacea and wrinkling caused by photodamage or conditions related to aging, hormonal imbalances, hyper-pigmentation, melasma, keratosis, dandruff, or the like. The compositions are also useful for removing biofilms from contact lenses. The potential use of such compositions in wound treatment is neither taught nor suggested.

The technical problem underlying the invention is to provide a wound-treatment composition with a favorable cleaning effect which is suitable for cleaning wounds, decontamination of body surfaces, dissolving incrustations and removing dressings and similar applications, which in combination with a biocide has a curative effect on wound-healing and which is substantially free of undesirable side effects.

This object is achieved by the composition according to claim 1. Preferred embodiments are given in the sub-claims.

The invention therefore relates to a composition for use as a wound-treatment agent which comprises (a) at least one silicate, (b) at least one carbonate, (c) at least one gluconate, and (d) at least one sulfate.

The compositions according to the invention may further comprise (e) at least one salt selected from alkali metal and/or alkaline earth metal sulfates, borates, bromides, citrates, acetates and lactates. The salt should not interfere with the biological activity of the composition. When other materials are present, the salt should not degrade those materials or interfere with their properties or biological activity. In other words, the salt should be inert with respect to the other components.

The compositions according to the invention show a remarkable and unexpected effect especially when used in aqueous solution as a rinsing agent for cleaning wounds. It has been surprisingly found that pretreatment of wounds with a composition according to the invention has a beneficial and synergistic effect on the traditional treatment of wounds with antibiotics or oxidizing agents in that it makes the latter treatment more effective.

The compositions according to the invention are therefore especially suitable as rinsing or soaking agents for cleaning and decontaminating surfaces of a wound, for removing biofilms from wound and other body surfaces, for dissolving incrustations or scabs from wound and other body surfaces and as dissolving agents for dressings.

Preferably, said at least one silicate is an alkali metal silicate selected from the group consisting of sodium or potassium metasilicate and sodium or potassium orthosilicate, and mixtures thereof, of which sodium metasilicate, in particular in the form of its pentahydrate is most preferred.

Preferably, said at least one carbonate is selected from the group consisting of sodium carbonate, sodium sesquicarbonate, sodium bicarbonate and mixtures thereof, of which sodium carbonate is most preferred.

Preferably, said at least one gluconate is selected from the group consisting of ammonium gluconate, lithium gluconate, sodium gluconate, sodium starch gluconate, potassium gluconate, ammonium acid gluconate, sodium acid gluconate, lithium acid gluconate, potassium acid gluconate, ammonium D-gluconate, lithium D-gluconate, sodium D-gluconate, potassium D-gluconate, gluconic acid, gluconic D-acid, gluconic L-acid, ammonium L-gluconate, lithium L-gluconate, sodium L-gluconate, potassium L-gluconate, calcium gluconate, calcium acid gluconate, calcium D-gluconate, calcium L-gluconate and mixtures thereof, of which sodium gluconate and potassium gluconate are most preferred.

Preferably, said at least one sulfate is selected from the group consisting of potassium aluminum sulfate, sodium sulfate, potassium sulfate, lithium sulfate, ammonium sulfate, magnesium sulfate, strontium sulfate, aluminum sulfate and mixtures thereof, of which potassium aluminum sulfate is most preferred.

The at least one salt defined in (e) may be a single salt material or a mixture of two or more salts alone. When the carrier matrix contains a mixture of salts, those salts are preferably present in equal amounts, e.g., a mixture of two salts in a 1:1 ratio.

Specific examples of suitable salts include, but are not limited to, sodium acetate, sodium bicarbonate, sodium borate, sodium bromide, sodium carbonate, sodium chloride, sodium citrate, sodium fluoride, sodium gluconate, sodium sulfate, calcium chloride, calcium lactate, calcium sulfate, potassium sulfate, tripotassium phosphate, potassium chloride, potassium bromide, potassium fluoride, magnesium chloride, magnesium sulfate and lithium chloride. Preferred salts are the inorganic salts, such as the Group 1 or 2 metal sulfates and chlorides. A particularly preferred salt, because of their low cost, is sodium chloride. Sodium chloride maybe substantially pure or in the form of rock salt, sea salt, or dendrite salt. Of these, Reef Crystals synthetic sea salt (Aquarium Systems, Inc.) is most preferred.

Silicates in the meta-forms of pentahydrates (e.g. Na₂SiO₃.5H₂O) are known in the art of cleaning technology as good builders with high pH, buffering capacity and dirt removal. Meta-silicates are known to prevent Ca precipitation. Silicates are unique among the oxy-anions, the common ionic 3D raster of SiO₄ is the tetrahedral.

Carbonates, exemplified by soda (Na₂CO₃), are known as effective builders with a high pH and effective buffering capacity. In very hard water precipitation may occur due to Ca and/or Mg carbonates. Soda performs well in dirt removal. Therefore, it is an active component in removal of extracellular Polymeric Substances (xPS) and soft not fully closed scaling.

Gluconates, exemplified by potassium gluconate, are known sequestrants. Combined with additives, such as sodium carbonates and/or meta-silicates, gluconates are active in scale removal from surfaces, including the skin.

Sulfates, exemplified by sodium sulfate, are known for their coagulation action.

In still a further aspect of the invention the compositions additionally comprise an emulsifying agent, a surfactant, a thickening agent, or a mixture thereof.

In a preferred embodiment of the invention the compositions further comprise a physiologically acceptable carrier, preferably selected from liposomes, solutions, in particular aqueous solutions, creams, emollients, ointments, gels, solid formulations and liquid formulations, or diluent.

In a most preferred embodiment of the invention the compositions are preferably used in aqueous solution at a pH which is preferably in the range of about 7.5 to about 9.

As stated before, the compositions according to the invention are especially suitable as rinsing or soaking agents for cleaning and decontaminating surfaces of a wound, for removing biofilms from wound and other body surfaces, for dissolving incrustations or scabs from wound and other body surfaces and as dissolving agents for dressings.

Without wanting to be bound to any scientific theory, the inventor believes that the surprising effects of the claimed compositions, in particular in cleaning surfaces of a wound and removing biofilms including glycocalyx produced by some bacteria, epithelia and other cells, may be explained as follows.

As biofilm formation starts from the first minute in aqueous environment, the first step is the formation of a conditioning film of organic molecules. After initial attachment, planktonic bacteria adhere irreversibly to the surface and proliferate to transform into bacterial micro-colonies. Subsequently bacteria generate a coating of xPS, which is essential for the development of the architecture of any biofilm matrix; it provides a framework into which microbial cells are inserted. Confocal laser scanning microscopy indicates that micro-colonies within a biofilm are three-dimensional structures of mushroom-like bacterial growth with water channels running between them for a constant supply of nutrients. The most part of the biofilm, generally around 97%, consists of water. The microbial cells form only about 2-5 % of the biofilm.

Briefly, according to the present invention, two compounds are selected for their builder activity, a meta-silicate and a carbonate. Both compounds creep behind the biofilm for loosening the xPS from the skin, and actually from the organic molecular layer on the skin. Together with two specific salts known for their coagulation and combined scale removing capabilities, a gluconate and a sulfate, the biofilm and existing scaling are removed with cleansing water.

Both the meta-silicate and carbonate are anionic in their action consisting of absorption to the xPS lowering the binding forces to their substrate. Bacteria in the xPS have positively charged cell walls as shown by Mera and Beveridge, J. Bacteriol 175 7:1936-1945 (1993). The original double layer of positive and negative molecules is loosened from the substrate by the meta-silicate and carbonate as they are negatively charged and creep under the xPS. Both meta-silicate and carbonate show to have synergistic action resulting in better cleaning as should be expected from the individual salts. This is expressed in the zeta potential which is still at the same level at some distance from the substrate surface compared to the situation with the xPS stuck directly in the wound. Zeta potential is defined as the electrical potential at the substrate surface with respect to the bulk liquid = water, i.e. the zeta is higher at the surface and becomes lower when the distance between substrate and biofilm or dirt increases. After addition of a composition according to the invention on the wound, it will seek the lowest potential energetic level. This basic rule in chemistry and physics is reflected in the situation of loosened and coagulated/encapsulated biofilm provided that the dosed concentrations are at the correct levels. In the end, the negatively charged organic layer or substrate is replaced by a negatively charged layer of the anionic salts composition of the invention.

In a further aspect of the present invention the composition comprises, in weight percent:
a) from about 0.1% to about 2.5%, preferably about 0.5% to about 2.0%, of a meta-silicate, preferably sodium meta-silicate;
b) from about 0.1% to about 2.0%, preferably from about 0.5% to about 1.5%, of a carbonate, preferably sodium carbonate;
c) from about 0.1% to about 1.0%, preferably from about 0.3% to about 0.7%, of a gluconate, preferably sodium gluconate;
d) from about 0.1% to about 1.0%, preferably from about 0.3% to about 0.7% of a sulfate, preferably potassium aluminum sulfate;
e) optionally, from about 0.1% to about 1.0%, preferably from about 0.3% to about 0.7% of a salt as defined herein, preferably sodium chloride, more preferably synthetic sea salt.

Preferably, the total percentage of ingredients in the composition is < 5 wt.%.

A particularly preferred composition according to the invention comprises:
- sodium metasilicate pentahydrate (PQ Silicas B.V., Netherlands), 1.83 wt.%;
- sodium carbonate (Tata Chemicals Europe, UK), 1.43 wt.%;
- sodium gluconate 99% (Jungbunzlauer S.A., France), 0.5 wt.%;
- potassium aluminum sulfate >99% (Kaliumaluin solid, Caldic Belgium N.V.), 0.5 wt.%;
- synthetic seasalt, containing 58% NaCl (Reef Crystals synthetic seasalt, Aquarium Systems, Inc.), 0.5 wt.%;
- in an aqueous solution, adding a suitable acid, for example citric acid or acetic acid or hydrochloric acid to adjust the pH to 7.5 to 9, in particular to about 8.

The concentration of the aqueous composition according to the invention for use as a wound-treatment agent may vary between ranges which are known in the art or can be derived from commercial wound-treatment agents, but is preferably in a dilution of about 1:200, i.e. 50 ml of stock solution in 10 liters of water.

The composition for use as a wound-treatment agent can principally be used in any form of preparation known and suitable for wound treatment, which are ointments, tinctures, sprays, rinsing solutions or a washing or shower gel for wound treatment, as a moisturizing gel or as a moist wound covering, as a dissolving gel for dissolving incrustations or scabs from body surfaces or wounds or for removing dressings and for changing moist dressings, and for treating burns and skin transplants.

Preferably, the compositions according to the invention are used as wound-treatment agent in the form of washing solutions, washing gels or gel-like wound coverings. For example, a wound-rinsing solution consists of 98 percent by weight of water, preferably 99.5 percent by weight of water.

When used in the form of a wound gel, a preferred composition contains 1 to 15 percent by weight of glycerine and 0.2 to 5 percent by weight of hydroxyethyl cellulose, especially 5 to 12 percent by weight of glycerine and 0.2 to 3 percent by weight of hydroxyethyl cellulose.

When the composition for use as a wound-treatment agent is used in the form of a wound gel, it contains the additives usually used in medical or cosmetic gels. For the compositions in accordance with the invention for use as a wound-treatment agent, additives of 1 to 15 percent by weight of glycerine and 0.2 to 5 percent by weight of hydroxyethyl cellulose, especially 5 to 12 percent by weight of glycerine and 0.2 to 3 percent by weight of hydroxyethyl cellulose, have proven to be advantageous for the mixtures as described above.

In all compositions in accordance with the present invention for use as wound-treatment agents, the additives and admixtures that are usually used for skin-cleaning, wound-cleaning and anti-infective agents can be present in addition to the mentioned components. Other preservatives, colorants, flavors and fragrances, binding agents, moisturizing or wetting agents, consistency regulators, solubilizers or the like can be mentioned, for example which can be added in the usual quantities.

It is preferable however to keep the number and concentration of the additives and admixtures as low as possible or to avoid the same entirely. It is understood that substances which lead to a reduced effect of the components already present or have a disadvantageous effect on the tissue to be cleaned shall be avoided as far as possible.

Either cleaned water or another aqueous solution as used otherwise in the field of medicine is suitable as a solvent in the compositions according to the invention for use as a wound-treatment agent. Suitable solvents are for example a saline or Ringer's solution. A saline solution of 0.4 to 1.2 percent of weight can be used for example. The saline or Ringer's solution is used especially preferably in a physiological dilution. In the case of solutions, the invention comprises both the solutions readily diluted and concentrates which need to be diluted prior to use.

The composition in accordance with the invention for use as a wound-treatment agent is especially suitable for treating chronic wounds, as occur for example in diabetics or bedridden patients. Diluted aqueous solutions can be used for rinsing or bathing wounds or for soaking and humidifying wound coverings. Adhering wound dressings could be removed easily from the wound by softening with the solution without damaging the wound tissue. When dressings are removed it often occurs that incrustations already formed are torn open again in an undesirable manner during the removal of the dressing, thus leading to delayed healing of the wound. The composition in accordance with the invention for use as a wound-treatment agent provides a remedy for this purpose too. When the composition in accordance with the invention for use as a wound-treatment agent is applied to the dressing, it penetrates and detaches the same from the incrustations on the wound, so that the dressing can be removed without damaging any incrustations already formed. At the same time, it is prevented that bacteria or the like are implanted in the wound.

The composition in accordance with the invention for use as a wound-treatment agent in gel form can be used as a moisturizing gel or moisture wound covering. Wound coatings can thus be detached, the wound can be cleaned and decontaminated. One application is the removal of scabs in patients' noses who are artificially respirated. The gel is also especially suitable for treating bums and skin transplants in order to keep the wound humid and smooth and to prevent forming breeding ground for bacteria and other germs. Application can be made directly on the wound or on a wound dressing. The gel layer reliably prevents recontamination of disinfected wounds by germs introduced from the outside.

The main field of application of the composition according to the invention for use as a wound-treatment agent is, as mentioned before, the cleaning and decontamination of wounds, wound conditioning and wound remediation. Main focus must be laid on the removal of wound coatings which delay the healing of wounds and form a breeding ground for the new settlement of pathogenic germs on non-infected wounds.

The composition according to the invention provides for the first time a composition for use as a wound-treatment agent which combines a favorable cleaning effect as a pretreatment of wounds with the traditional treatment of wounds with antibiotics or oxidizing agents, e.g. a peroxide ointment, in that it makes the latter treatment more effective, without leading to irritations or even destruction of the tissue. Wounds are reliably cleaned and decontaminated, odor formation is suppressed, and wound healing is promoted.

The invention will now be explained in further detail with reference to the following example.

### Example

In this example it is demonstrated that a preferred composition according to the invention, hereinafter named "Aqua Finesse" is effective in the treatment of biofilms in vitro and in chronic wounds.

Aqua Finesse had the same composition as the preferred composition disclosed in the general description:
- sodium metasilicate pentahydrate, 1.83 wt.%;
- sodium carbonate, 1.43 wt.%;
- sodium gluconate (99%), 0.5 wt.%;
- potassium aluminum sulfate (>99%), 0.5 wt.%;
- synthetic seasalt (containing 58% NaCl), 0.5 wt.%;
in an aqueous solution, to which citric acid was added to adjust the pH to 8.0.

The following strains of bacteria were tested on treatment with "Aqua Finesse":
- *Staphylococcus aureus,*
- *Staphylococcus epidermidis*
- *Enterococcus faecalis*
- *Proteus vulgaris*
- *Enterobacter cloacae*

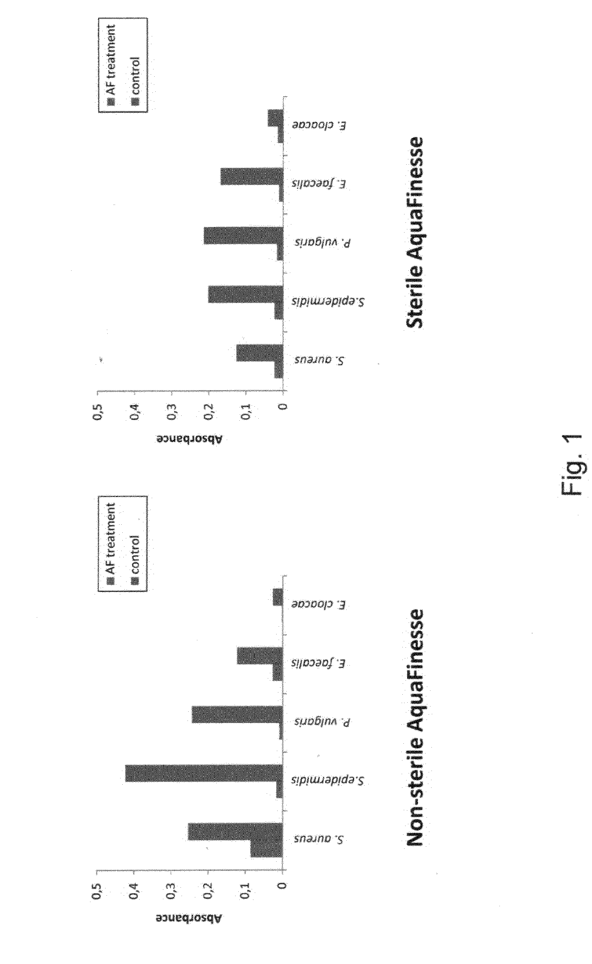

These bacteria are all to be found in open wounds. Many infections are polymicrobial. The results showed that "Aqua Finesse", both as a sterile and non-sterilized product, performs efficiently on the biofilms. See Figure 1

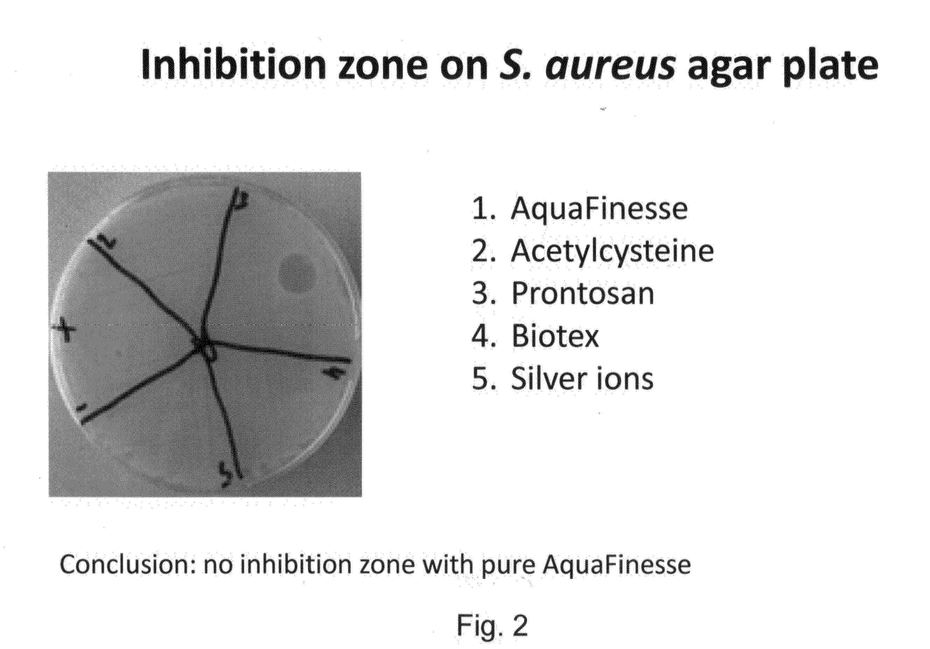

Pure "Aqua Finesse" was applied on S. aureus agar plate to determine its possible biocidal effect. However, no inhibition zone was found. See Figure 2.

A slime-test was performed with the above five bacteria strains and mixtures thereof, and with *Pseudomonas aeruginosa* at two different doses of "Aqua Finesse" to assess if it dissolved the "slime" layer of the biofilm. All these tests proved that "Aqua Finesse" had effect in removing the slime layer and making biofilms susceptible to antibiotics.

An MTT assay was performed with the separate bacteria biofilms and a biofilm of the mixture exposed to 3 different doses of "Aqua Finesse" for 5 and 30 minutes. An MTT assay was also performed to assess the effectiveness of treatment with a combination of "Aqua Finesse" and antibiotics. Afterwards a confocal laser scanner microscope was used. It was demonstrated that antibiotic treatment is more effective after pre-treatment with "AquaFinesse".

It was further surprising to note that the test with *S. aureus* showed a biofilm left, but it became susceptible for antibiotics. This bacteria is normally resistant against antibiotics. It was concluded that removing the outer layer of the biofilm may be sufficient for a successful treatment of the bacteria with antibiotics.

An MTT assay showed that the metabolic activity of the bacteria does not change when exposed to "Aqua Finesse" in different concentrations. Performing multiple slime-tests showed that treatment with "Aqua Finesse" partially opens and loosens biofilm slime and resulted in fewer biofilm clumps.

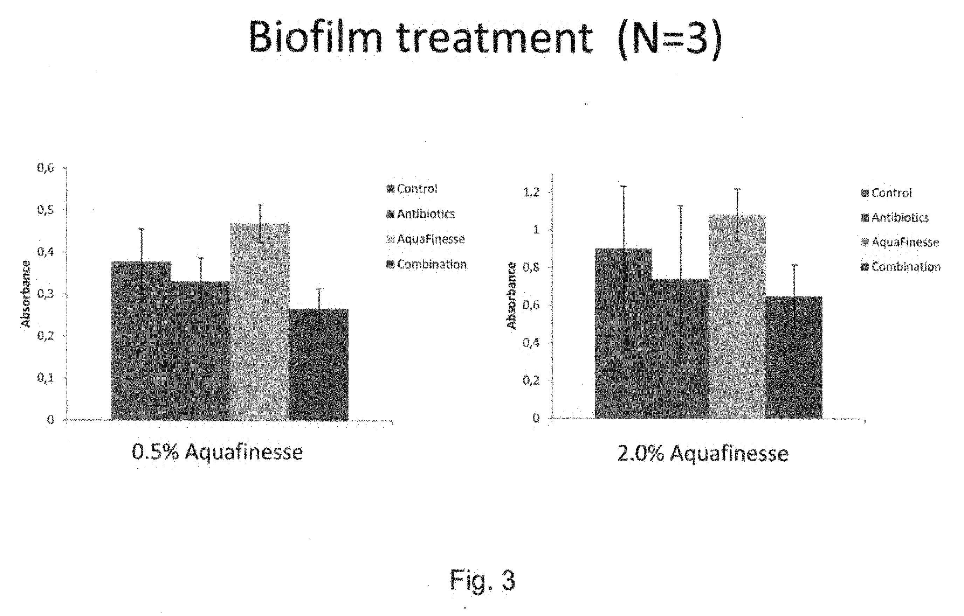

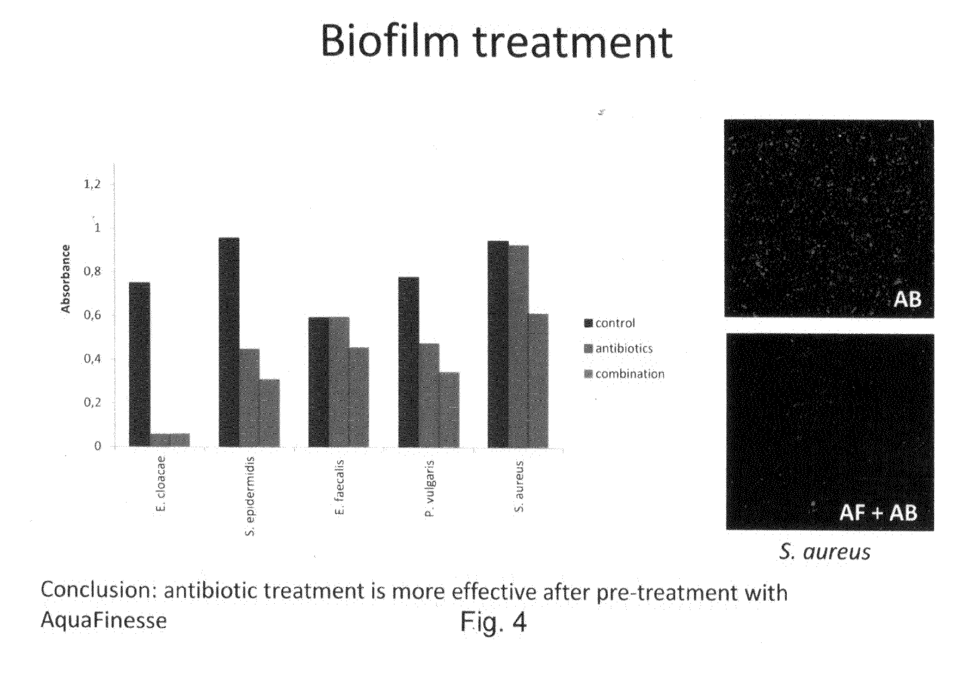

MTT assay analysis showed prophylactic use of "Aqua Finesse" to have no noticeable effect. Furthermore treatment of biofilms with only "Aqua Finesse" or antibiotics showed no significant improvement, whereas treatment with "Aqua Finesse" followed by antibiotic treatment showed significantly better results. See Figs. 3 and 4.

Confocal laser scanning microscopy showed that the biofilm thickness of the mixture of *S*. *aureus and Ps. Aeruginosa* treated only with "Aqua Finesse" decreased the least as compared to the control group. The biofilm exposed to antibiotics and the combination of "Aqua Finesse" and antibiotics showed a reduction in thickness. The CLSM confirmed that the combination of "Aqua Finesse" and antibiotics was the most effective against the biofilms by showing more dead bacteria, although it did not completely remove the biofilm. Although some biofilm was still present it was not attached to the surface anymore.

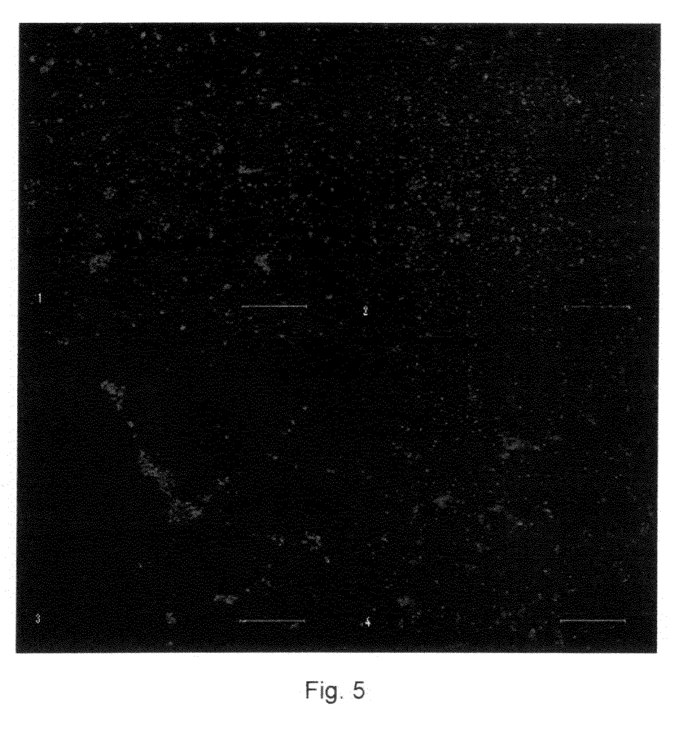

The mixed biofilm of *S. aureus* and *Pseudomonas* was used for the Confocal laser scanning microscopy (CLSM). The biofilm was tested in 4 different conditions. The first well was filled with an untreated biofilm of the mixture as control. In the second well antibiotics were added to the biofilm. "Aqua Finesse" was added to the third well. The fourth well was treated with a combination of "Aqua Finesse" and the antibiotics. Figure 5 shows the CLSM of the biofilm in the different conditions. The green dots show the living cells and the red dots show the death cells. The picture shows that "Aqua Finesse" in combination with the antibiotic kills many cells.

## Claims

1. Use of a composition comprising:
(a) at least one silicate,
(b) at least one carbonate,
(c) at least one gluconate, and
(d) at least one sulfate,
as a wound-treatment agent.

2. Use of a composition according to claim 1, wherein the composition further comprises (e) at least one salt selected from alkali metal and/or alkaline earth metal sulfates, chlorides, borates, bromides, citrates, acetates and lactates.

3. Use of a composition according to claim 2, wherein said at least one salt is sodium chloride.

4. Use of a composition according to any one of claims 1-3, wherein the composition is in the form of an aqueous solution having a pH of 7.5 - 9.

5. Use of a composition according to claim 1, wherein said at least one silicate is an alkali metal silicate selected from the group consisting of sodium or potassium metasilicate, and mixtures thereof.

6. Use of a composition according to claim 1, wherein said at least one carbonate is selected from the group consisting of sodium carbonate, sodium sesquicarbonate, sodium bicarbonate, and mixtures thereof.

7. Use of a composition according to claim 1, wherein said at least one gluconate is selected from the group consisting of ammonium gluconate, lithium gluconate, sodium gluconate, sodium starch gluconate, potassium gluconate, ammonium acid gluconate, sodium acid gluconate, lithium acid gluconate, potassium acid gluconate, ammonium D-gluconate, lithium D-gluconate, sodium D-gluconate, potassium D-gluconate, gluconic acid, gluconic D-acid, gluconic L-acid, ammonium L-gluconate, lithium L-gluconate, sodium L-gluconate, potassium L-gluconate, calcium gluconate, calcium acid gluconate, calcium D-gluconate, calcium L-gluconate, and mixtures thereof.

8. Use of a composition according to claim 1, wherein said at least one sulphate is selected from the group consisting of potassium aluminum sulfate, sodium sulfate, potassium sulfate, lithium sulfate, ammonium sulfate, magnesium sulfate, strontium sulfate, aluminum sulfate, and mixtures thereof.

9. Use of a composition according to claim 1 or 2, wherein the composition comprises (in wt. percent)
a) from about 0.1% to about 2.5%, preferably about 0.5% to about 2.0%, of a meta-silicate, preferably sodium meta-silicate;
b) from about 0.1% to about 2.0%, preferably from about 0.5% to about 1.5%, of a carbonate, preferably sodium carbonate;
c) from about 0.1% to about 1.0%, preferably from about 0.3% to about 0.7%, of a gluconate, preferably sodium gluconate;
d) from about 0.1% to about 1.0%, preferably from about 0.3% to about 0.7% of a sulfate, preferably potassium aluminum sulfate;
e) optionally, from about 0.1% to about 1.0%, preferably from about 0.3% to about 0.7% of a salt as defined herein, preferably sodium chloride, more preferably synthetic sea salt.

10. Use of a composition according to claim 1, wherein the composition further comprises an emulsifying agent, a surfactant, a thickening agent, or a mixture thereof.

11. Use of a composition according to claim 1, wherein the composition further comprises a physiologically acceptable carrier, preferably selected from liposomes, solutions, creams, emollients, ointments, gels, solid formulations and liquid formulations, or diluent.

12. Use of a composition according to claim 1, wherein the composition is in the form of a liquid, cream, oil, gel, fluid cream, lotion, emulsion or micro-emulsion.

13. Use of a composition according to any one of claims 1 to 11, wherein the composition is in the form of a wound-rinsing solution, which consists of at least 98 percent by weight of water.

14. Use of a composition according to any one of claims 1 to 11, wherein the composition is in the form of a wound gel, which contains 1 to 15 percent by weight of glycerine and 0.2 to 5 percent by weight of hydroxyethyl cellulose, especially 5 to 12 percent by weight of glycerine and 0.2 to 3 percent by weight of hydroxyethyl cellulose.

15. Use of a composition according to any one of claims 1 to 11, as a rinsing solution or a washing or shower gel for wound treatment, as a moisturizing gel or as a moist wound covering, as a dissolving gel for dissolving incrustations or scabs from body surfaces or wounds or for removing dressings and for changing moist dressings, and for treating burns and skin transplants.
